# EUROPEAN PATENT SPECIFICATION

(11) **EP 1 204 391 B1**
(45) Date of publication and mention of the grant of the patent: **13.10.2004**
(21) Application number: 00953325.8
(22) Date of filing: 15.08.2000
(51) Int. Cl.: A61F 13/02

(54) **EASY TO REMOVE ADHESIVE SHEETS**
LEICHT ABLÖSBARE KLEBESCHICHTEN
FEUILLES ADHESIVES FACILES A ENLEVER

(30) Priority: 16.08.1999 GB 9919368
(43) Date of publication of application: 15.05.2002
(73) Proprietor: JOHNSON & JOHNSON MEDICAL LIMITED., Edinburgh EH2 4NH (GB)
(72) Inventor: PATEL, Dharmendra, Skipton North Yorkshire BD23 2PB (GB); SILCOCK, Derek, Skipton North Yorkshire BD23 1QP (GB); CULLEN, Breda, Skipton North Yorkshire BD23 1HR (GB); LOWING, Paul Howard, Keighley, West Yorkshire BD21 2BZ (GB)
(74) Representative: James, Anthony Christopher W.P.
(86) International application number: PCT/GB2000/003146
(87) International publication number: WO 2001/012116

(56) References cited:
- DE-A- 3 316 635
- US-A- 4 822 670
- DATABASE WPI Section PQ, Week 198941 Derwent Publications Ltd., London, GB; Class P43, AN 1989-292815 XP002153006 & AU 29973 89 A (MOSS L), 17 August 1989 (1989-08-17)

## Description

The present invention relates to easy to remove adhesive sheets. The present invention also relates to medical adhesive tapes and wound dressings comprising such easy to remove adhesive sheets.

A number of medicinal applications utilise self-adhesive sheet materials for attachment to human skin. For example, self-adhesive tapes comprising a layer of pressure-sensitive adhesive on a backing sheet of polymeric film or fabric are used to secure catheters and other medical devices in position on the body of the patient.

Wound dressings are also frequently secured in position over a wound by the use of a backing sheet or tape coated with pressure-sensitive adhesive for application to the relatively undamaged skin of a patient around a wound. Preferred wound dressings are the so-called island wound dressings, which comprise a wound contacting layer of absorbent and/or therapeutic material applied to a microorganism-impermeable backing sheet. The backing sheet is coated with a layer of pressure-sensitive adhesive on the side proximate to the wound contacting layer, and the area of the backing sheet is greater than the area of the wound contacting layer, whereby an adhesive-coated margin of backing sheet extends beyond at least two sides of the wound contacting layer. In use, the adhesive-coated margin is secured to the relatively undamaged skin adjacent to the wound, such that the wound contacting layer faces the wound. Island-type wound dressings are described for example in GB-A-2 074 029, and are available for example from Johnson & Johnson under the Registered Trade Mark ELASTOPLAST.

It is also known for example from US-A-4 695 277 or GB-A-845 841 to provide adhesive patches for transdermal administration of medicaments comprising a reservoir of medicament that is held in contact with the skin of a patient by an adhesive-coated sheet extending around the reservoir.

A drawback of conventional medical adhesive tape, adhesive wound dressings and adhesive transdermal patches is that removal of the adhesive sheet by pulling it off the skin can cause discomfort to the patient. Moreover, the force required to pull a self-adhesive wound dressing away from a wound can traumatise the wound and retard wound healing.

The above difficulties can be alleviated by providing a less strongly adherent layer of adhesive on the adhesive sheet. However, of course, this also results in less secure adhesion of the tape or wound dressing to the skin in use.

It would be desirable to provide self-adhesive sheets that can be attached to and removed from human skin without these disadvantages. Accordingly, it is an object of the present invention to provide adhesive sheets, especially for use in medical applications, which combine strong adhesion with ease of removal.

The present invention provides an adhesive sheet comprising: a backing layer; a layer of adhesive applied to a first side of the backing layer for adhering the adhesive sheet to a surface; and an elongate conduit for a fluid provided in or on said first side of the backing layer, wherein the conduit is provided with an inlet for introducing fluid into the conduit while the adhesive sheet is adhered to the surface to assist removal of the adhesive sheet from the surface.

Preferably, the backing layer is substantially impermeable to liquids and microorganisms. For example, the backing layer preferably comprises a substantially continuous film or sheet.

Preferably, the backing layer is semipermeable. That is to say, the backing layer is permeable to water vapour, but not permeable to aqueous liquids. Suitable continuous conformable backing layers will have a moisture vapour transmission rate (MVTR) of the backing layer alone of 300 to 5000 g/m²/24hrs, preferably 500 to 2000 g/m²/24hrs at 37.5 °C at 100% to 10% relative humidity difference. The backing layer thickness is preferably in the range of 10 to 1000 micrometers, more preferably 100 to 500 micrometers.

Suitable polymers for forming the backing sheet include polyurethanes and polyalkoxyalkyl acrylates and methacrylates such as those disclosed in GB-A-1280631. Preferably, the backing layer comprises a continuous layer of a high density blocked polyurethane foam that is predominantly closed cell. A suitable backing sheet material is the polyurethane film available under the Registered Trade Mark ESTANE 5714F.

The layer of adhesive applied to a first side of the backing layer is preferably a pressure-sensitive adhesive. Preferably, the layer of adhesive extends over the whole of the first side of the backing layer in uniform fashion. However, the layer of adhesive may be interrupted or patterned. Preferably, the layer of adhesive is moisture vapour transmitting. More preferably, the pressure sensitive adhesive itself is moisture vapour transmitting.

The adhesive is preferably water-releasable. Preferred pressure-sensitive adhesives include the acrylate ester copolymer, polyvinyl ethyl ether and polyurethane based pressure-sensitive adhesives conventionally used in adhesive wound dressings and medical tapes, and described for example in GB-A-1280631. A particularly preferred adhesive is a polyether polyol adhesive that is available under the Registered Trade Mark LEVAGEL. The basis weight of the adhesive layer is preferably 20 to 250 g/m², and more preferably 50 to 150 g/m².

The present invention provides ease of removal of the adhesive coated sheet by the additional feature of a conduit provided on or in the first side of the backing layer to which can be supplied a fluid to reduce the adherency of the adhesive and thereby assist removal of the adhesive sheet in use. For example, the fluid could be a liquid that is released from the conduit into the adhesive layer, where it dissolves or weakens the adhesive. Preferably, the liquid is an aqueous liquid, such as a sterile saline solution. In other embodiments, the fluid could be a liquid or a gas at superatmospheric pressure that inflates the conduit and thereby mechanically assists removal of the adhesive sheet.

The conduit preferably extends transversely across the first side of the backing layer. It may be recessed or embedded in the first side. The conduit may have any cross-sectional shape. The conduit is preferably collapsible to a flat shape when not filled with liquid or gas, for example when the adhesive sheet is pressed onto a surface. The mean cross-sectional area of the conduit when filled with liquid or gas is preferably from 0.05 to 5.0 mm².

Preferably the conduit comprises a tube applied to the first side of the backing layer. The tube may be adhered to the backing layer by the adhesive layer, and further adhesive may be provided on the surface of the tube to render the tube adherent. The diameter of the tube is preferably from 0.5 to 6.0 mm, more preferably 1.0 to 3.0 mm. Preferably, the tube is rendered liquid-permeable by perforations, which are preferably spaced along the tube at intervals of 1 to 30 mm, more preferably 2 to 10 mm in a random or ordered fashion. The hole size of the perforations is preferably from 0.1 to 1.5 mm², preferably from 0.2 to 1.0 mm².

Preferably, the tube extends in an arcuate, serpentine or branched fashion across the backing layer, or forms part of a network of tubes extending transversely across the adhesive-coated sheet. In other preferred embodiments, the tube extends around the adhesive sheet close to the margin of the adhesive sheet, for example 5 to 20 mm from the edge of the adhesive sheet. This latter arrangement is especially suitable when the adhesive sheet forms the backing sheet of an island-type wound dressing.

The tube is preferably a polymer tube, such as a perforated polyolefin tube. The tube can be rigid or collapsible and/or flexible. The tube may be formed from any physiologically acceptable polymer, including polyolefins, rubber, neoprene, silicone elastomer, TYGON (Registered Trade Mark), VERERNET (Registered Trade Mark), NORTON (Registered Trade Mark), polyvinyl chloride, polyurethane, polytetrafluoroethylene, VISKING (Registered Trade Mark), borosilicate, or regenerated cellulose. The tube may be perforated by any conventional means, e.g. electric discharge, to render it liquid permeable.

The adhesive sheet further comprises an inlet for introducing a fluid into the conduit. For example, the preferred tube may extend outwardly of the adhesive sheet to terminate in an inlet coupling, for example, a coupling suitable for connection to a source of sterile saline solution such as a septum or a Luer lock syringe coupling.

A method of use of an adhesive sheet according to the present invention comprises the steps of: adhering the backing layer to a surface by said adhesive layer; and, when it is desired to remove the backing layer from the surface, introducing a liquid or a gas under pressure into the conduit to assist removal of the backing layer from the surface.

Preferably, the conduit is substantially empty (apart from air at atmospheric pressure) prior to the said step of introducing.

In a further aspect, the present invention provides an adhesive sheet comprising: a backing layer; a layer of adhesive applied to a first side of the backing layer for adhering the adhesive sheet to a surface; and an elongate reservoir containing a liquid release agent provided in or on said first side of the backing layer, wherein the reservoir can be ruptured to release the liquid therefrom into the adhesive layer while the adhesive sheet is adhered to the surface to assist removal of the adhesive sheet from the surface.

Preferably, the reservoir is provided with a release strip such as a pull strip or a thread extending outwardly of the backing layer to release the liquid from the reservoir when the release strip is pulled. For example, the reservoir preferably comprises a liquid-filled impermeable tube equipped with a tear strip that can be pulled from outside the edge of the dressing for releasing the liquid.

The present invention also provides a medical adhesive tape for attachment to human skin, comprising an adhesive sheet according to the present invention as hereinbefore defined.

The present invention also provides a wound dressing comprising an adhesive sheet according to the present invention as hereinbefore defined. Preferably, the wound dressing is an island-type dressing further comprising a layer of absorbent material provided over a part of the adhesive-coated side of the backing layer. More preferably, an adhesive-coated margin of the backing layer extends around all sides of the layer of absorbent material.

The present invention also provides a patch for the transdermal administration of a medicament comprising a reservoir of the medicament and an adhesive sheet according to the invention.

Preferably, the adhesive sheets according to the present invention further comprise one or more removable protective sheets over the layer of adhesive and the conduit and/or reservoir to protect the layer of adhesive before use. Preferably, the cover sheets comprise release-coated paper, such as silicone-coated paper.

Preferably, the adhesive sheets according to the present invention are sterile, for example, they may be sterilised by gamma-irradiation. Preferably, the adhesive sheets according to the present invention are packaged in a microorganism-impermeable container prior to use.

Certain embodiments of the present invention will now be described further, by way of example, with reference to the accompanying drawings, in which:
Figure 1: shows a bottom plan view of an island-type wound dressing incorporating an adhesive sheet according to the present invention;
Figure 2 : shows schematic sectional views through an adhesive sheet according to a second embodiment of the present invention incorporating a liquid reservoir and a pull strip: (a) before release of liquid form the reservoir, and (b) during release of liquid from the reservoir; and
Figure 3 shows a schematic partially cut-away partial view of the liquid reservoir and tear strip of the embodiment of Figure 2.

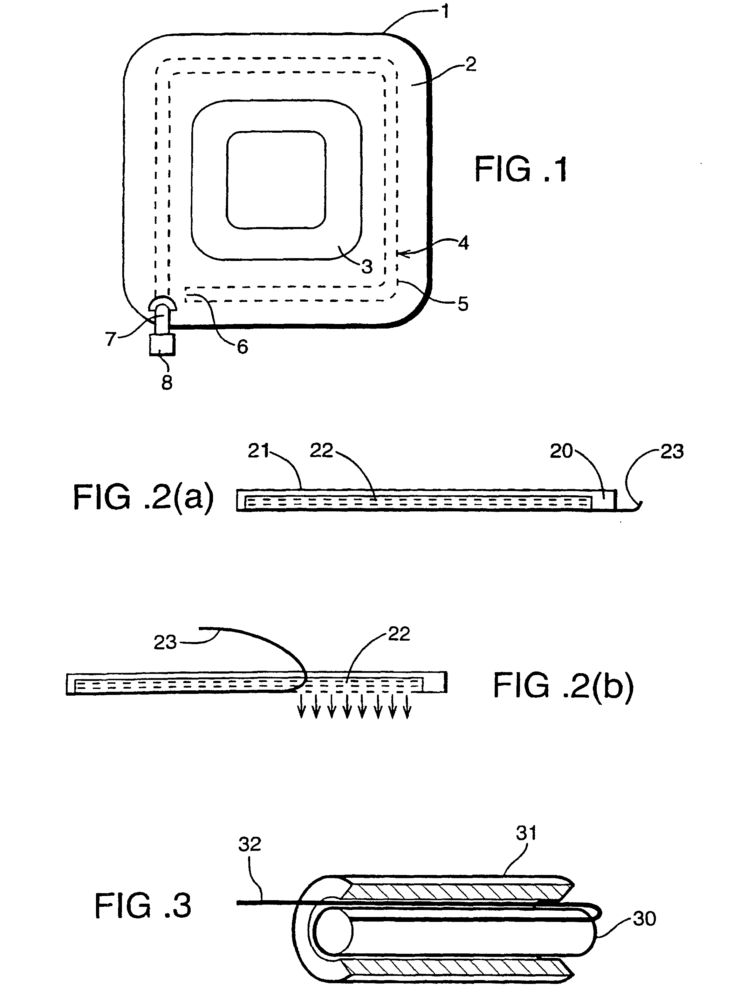

Referring to Figure 1, the island-type would dressing comprises a backing sheet 1 formed from a substantially closed-cell microporous polyurethane foam having a water vapour transmission rate of 1200-1300 g/m²/24hr at 37.5°C at 100% to 10% relative humidity difference. The backing sheet thickness is approximately 250 micrometers, and the backing sheet is substantially impermeable to liquids and microorganisms. A suitable backing sheet material is the polyurethane film available under the Registered Trade Mark ESTANE 574F (BF Goodrich).

The backing sheet 1 is provided on its lower surface with a substantially continuous layer 2 of pressure-sensitive adhesive. The adhesive is a water vapour permeable, pressure-sensitive adhesive that is available under the Registered Trade Mark LEVAGEL. It is applied by spray coating or transfer coating. The adhesive is applied in an amount of 90-110 g/m².

A wound contacting layer 3 is provided in the form of an island centred on the adhesive-coated backing sheet. The wound contacting layer comprises a layer of open cell hydrophilic polyurethane foam approximately 2 mm thick of the kind described in EP-A-0541391. The wound contacting layer 3 is secured to the backing sheet by the adhesive layer 2. The wound facing surface of the wound contacting layer 3 is itself substantially free of adhesive.

The island wound dressing further comprises means to assist removal of the wound dressing with reduced discomfort. This means comprises a tube 4 of polymer film extending around the margin 5 of the dressing. The diameter of the tube is about 2 mm. The tube 4 is perforated throughout its length at intervals of about 5 mm, the perforation size being about 0.5 mm², and closed at a distal end 6. A first end 7 of the tube 5 extends outwardly of the backing layer 1, and terminates in a standard syringe connection 8 for connecting the tube to a source of physiological saline. The tube 5 is attached to the backing layer by the layer of adhesive 2, and further adhesive coats the tube 5 on the opposite surface of the tube.

The island wound dressing is further provided with two silicone-coated release papers (not shown) covering the whole lower surface of the dressing, including the adhesive-coated margin of the backing layer and the wound contacting island. The wound dressing is packaged in a microorganism-impermeable pouch (not shown) and sterilized by gamma irradiation.

In use, the release-coated cover sheets are removed, and the island dressing is applied to a wound by adhering the adhesive-coated margins of the dressing to relatively undamaged skin around the wound such that the absorbent wound-contacting island is in contact with the wound. The dressing is applied to the wound by applying pressure to the adhesive-coated margin, and the tube 5 is empty and collapsed while the dressing remains applied to the wound.

When it is desired to remove the dressing from the wound, the inlet 8 is connected to a source of physiological saline, and the saline is then injected into the tube 5. The injected saline spreads into the adhesive layer 2 from the perforations in the tube, reducing the adherency of the layer and making it easier to remove the dressing.

Referring to Figure 2, this embodiment of the invention comprises a backing layer 20 similar to the backing layer 1 of Figure 1, and a similar pressure-sensitive adhesive layer 21. However, a reservoir of sterile physiological saline 22 is provided on the adhesive layer 21 side of the backing sheet 20 and. A tear strip 23 in the reservoir extends outwardly of the backing layer 20, and can be pulled to release the saline from within the reservoir when it is desired to remove the adhesive sheet from the skin of a patient.

A preferred arrangement of the reservoir and the tear strip is shown in more detail in Figure 3. In this embodiment, the reservoir 30 is in the form of a liquid-filled closed tube. A tear strip 32 is bonded to the tube 30 along its length and then is folded back along the tube. The tear strip 32 and tube 30 are encased in a concentric, perforated tube 31 that guides and protects the tear strip 32 and tube 30.

The above embodiments have been described by way of example only. Many other embodiments falling within the scope of the accompanying claims will be apparent to the skilled reader.

## Claims

1. An adhesive sheet comprising: a backing layer (1), a layer of adhesive (2) applied to a first side of the backing layer (1) for adhering the adhesive sheet to a surface; and an elongate conduit (4) for a fluid provided in or on said first side of the backing layer, wherein the conduit (4) is provided with an inlet (8) for introducing fluid into the conduit (4) while the adhesive sheet is adhered to the surface to assist removal of the adhesive sheet from the surface.

2. An adhesive sheet according to claim 1, wherein the backing layer (1) is substantially impermeable to liquids and microorganisms.

3. An adhesive sheet according to claim 1 or 2, wherein the backing layer (1) comprises a substantially liquid-impermeable polymer film.

4. An adhesive sheet according to any preceding claim, wherein the backing layer (1) comprises a polyurethane or a polyolefin sheet.

5. An adhesive sheet according to any preceding claim, wherein said layer of adhesive (2) extends over the whole of said first side of the backing layer.

6. An adhesive sheet according to any preceding claim, wherein said layer of adhesive (2) is patterned or interrupted.

7. An adhesive sheet according to any preceding claim, wherein said adhesive is a pressure-sensitive adhesive.

8. An adhesive sheet according to claim 7, wherein said pressure-sensitive adhesive comprises a polyurethane or a polyacrylate.

9. An adhesive sheet according to any preceding claim, wherein the adhesive layer (2) and the conduit (4) are covered by one or more release-coated cover sheets.

10. An adhesive sheet according to any preceding claim, wherein said conduit (4) comprises a liquid- and gas- impermeable tube extending transversely across said first side of said backing layer (1).

11. An adhesive sheet according to any preceding claim, wherein said conduit (4) comprises a liquid-permeable tube extending transversely across said first side of said backing layer (1).

12. An adhesive sheet according to claim 11, wherein said liquid-permeable tube comprises a perforated tube.

13. An adhesive sheet according to any one of claims 10 to 12, wherein said tube extends transversely across said first side of said backing layer (1) in an arcuate, serpentine or branched fashion.

14. An adhesive sheet according to any one of claims 10 to 13, comprising a network of interconnected tubes extending transversely across said first side of said backing layer (1).

15. An adhesive sheet comprising: a backing layer (20); a layer of adhesive (21) applied to a first side of the backing layer (20) for adhering the adhesive sheet to a surface; and an elongate reservoir (22) containing a liquid release agent provided in or on said first side of the backing layer (20), wherein the reservoir (22) can be ruptured to release the liquid therefrom into the adhesive layer while the adhesive sheet is adhered to the surface to assist removal of the adhesive sheet from the surface.

16. An adhesive sheet according to claim 15, wherein the reservoir (22) is provided with a release strip (23) extending outwardly of the backing layer to release the liquid from the reservoir when the release strip is pulled.

17. A medical adhesive tape for attachment to human skin, comprising an adhesive sheet according to any one of claims 1 to 16.

18. A wound dressing comprising an adhesive sheet according to any one of claims 1 to 16.

19. A wound dressing according to claim 18, further comprising a layer of absorbent material (3) provided over a part of said first side of said backing layer (1).

20. A wound dressing according to claim 18, wherein the backing layer (1) provides an adhesive-coated margin (5) around all sides of the layer of absorbent material (3).

21. A patch for the transdermal administration of a medicament comprising a reservoir of the medicament and an adhesive sheet according to any one of claims 1 to 16.

## Patentansprüche

1. Haftlage, umfassend eine Stützschicht (1), eine Haftschicht (2), aufgetragen auf eine erste Seite der Stützschicht (1) zur Haftung der Haftschicht an eine Oberfläche; ein verlängertes Rohr (4) für ein in- oder auf der ersten Seite der Stützschicht zur Verfügung gestelltes Fluid, wobei das Rohr (4) mit einem Einlass (8) zur Einführung von Fluid in das Rohr (4) ausgestattet ist, während die Haftlage an der Oberfläche angeheftet ist, um die Entfernung der Haftlage von der Oberfläche zu unterstützen.

2. Haftlage nach Anspruch 1, wobei die Stützschicht (1) im Wesentlichen undurchlässig gegenüber Flüssigkeiten und Mikroorganismen ist.

3. Haftlage nach Anspruch 1 oder 2, wobei die Stützschicht (1) einen im Wesentlichen flüssigkeitsundurchlässigen Polymerfilm umfasst.

4. Haftlage nach einem der vorangehenden Ansprüche, wobei die Stützschicht (1) eine Polyurethan- oder Polyolefinlage umfasst.

5. Haftlage nach einem der vorangehenden Ansprüche, wobei die Haftschicht (2) sich über die gesamte erste Seite der Stützschicht erstreckt.

6. Haftlage nach einem der vorangehenden Ansprüche, wobei die Haftschicht (2) gemustert oder unterbrochen ist.

7. Haftlage nach einem der vorangehenden Ansprüche, wobei das Haftmittel ein druckempfindliches Haftmittel ist.

8. Haftlage nach Anspruch 7, wobei das druckempfindliche Haftmittel ein Polyurethan oder Polyacrylat umfasst.

9. Haftlage nach einem der vorangehenden Ansprüche, wobei die Haftschicht (2) und das Rohr (4) mit einer oder mehreren freisetzungsbeschichteten Decklagen bedeckt ist.

10. Haftlage nach einem der vorangehenden Ansprüche, wobei das Rohr (4) eine flüssigkeits- und gasundurchlässige Röhre umfasst, die sich transversal über die erste Seite der Stützschicht (1) erstreckt.

11. Haftlage nach einem der vorangehenden Ansprüche, wobei das Rohr (4) eine flüssigkeitsdurchlässige Röhre umfasst, die sich transversal über die erste Seite der Stützschicht (1) erstreckt.

12. Haftlage nach Anspruch 11, wobei die flüssigkeitsdurchlässige Röhre eine perforierte Röhre umfasst.

13. Haftlage nach einem der Ansprüche 10 bis 12, wobei die Röhre sich transversal über die erste Seite der Stützschicht (1) in bogenförmiger, gewundener oder verzweigter Weise erstreckt.

14. Haftlage nach einem der Ansprüche 10 bis 13, umfassend ein Netzwerk an verbundenen Röhren, die sich transversal über die erste Seite der Stützschicht (1) erstrecken.

15. Haftlage umfassend eine Stützschicht (20); eine Haftschicht (21), aufgetragen auf die erste Seite der Stützschicht (20) zur Haftung der Haftlage an eine Oberfläche; und ein verlängertes Reservoir (22), enthaltend ein flüssigkeitsfreisetzendes Mittel, zur Verfügung gestellt in oder auf der ersten Seite der Stützschicht (20), wobei das Reservoir (22) aufgerissen werden kann, um Flüssigkeit daraus in die Haftschicht freizusetzen, während die Haftlage an der Oberfläche anhaftet, um die Entfernung der Haftlage von der Oberfläche zu unterstützen.

16. Haftlage nach Anspruch 15, wobei das Reservoir (22) mit einem Freisetzungsstreifen (23) ausgestattet ist, der sich von der Stützschicht nach außen erstreckt, um die Flüssigkeit in dem Reservoir freizusetzen, wenn der Freisetzungsstreifen gezogen wird.

17. Medizinisches Haftpflaster zur Befestigung an menschlicher Haut, umfassend eine Haftlage gemäß einem der Ansprüche 1 bis 16.

18. Wundverband, umfassend eine Haftlage gemäß einem der Ansprüche 1 bis 16.

19. Wundverband gemäß Anspruch 18, ferner umfassend eine Lage an absorbierendem Material (3), die über einen Teil der ersten Seite der Stützschicht (1) zur Verfügung gestellt wird.

20. Wundverband nach Anspruch 18, wobei die Stützschicht (1) über einen haftmittelbeschichteten Rand (5) um alle Seiten der Schicht aus absorbierendem Material (3) verfügt.

21. Pflaster zur transdermalen Verabreichung eines Medikaments, umfassend ein Reservoir für das Medikament und eine Haftlage gemäß einem der Ansprüche 1 bis 16.

## Revendications

1. Feuille adhésive comprenant : une couche support (1) ; une couche d'adhésif (2) appliquée à une première face de la couche support (1) pour faire adhérer la feuille adhésive à une surface ; et un conduit allongé (4) pour un fluide placé dans ou sur ladite première face de la couche support ; dans laquelle le conduit (4) est muni d'une entrée (8) pour introduire un fluide dans le conduit (4), tandis que la feuille adhésive adhère à la surface pour aider à l'enlèvement de la feuille adhésive de la surface.

2. Feuille adhésive selon la revendication 1, dans laquelle la couche support (1) est essentiellement imperméable aux liquides et aux micro-organismes.

3. Feuille adhésive selon la revendication 1 ou 2, dans laquelle la couche support (1) comprend un film polymère essentiellement imperméable aux liquides.

4. Feuille adhésive selon l'une quelconque des revendications précédentes, dans laquelle la couche support (1) comprend une feuille de polyuréthane ou de polyoléfine.

5. Feuille adhésive selon l'une quelconque des revendications précédentes, dans laquelle ladite couche d'adhésif (2) s'étend sur l'ensemble de ladite première face de la couche support.

6. Feuille adhésive selon l'une quelconque des revendications précédentes, dans laquelle ladite couche d'adhésif (2) est formée selon un motif ou interrompue.

7. Feuille adhésive selon l'une quelconque des revendications précédentes, dans laquelle ledit adhésif est un adhésif sensible à la pression.

8. Feuille adhésive selon la revendication 7, dans laquelle ledit adhésif sensible à la pression comprend un polyuréthane ou un polyacrylate.

9. Feuille adhésive selon l'une quelconque des revendications précédentes, dans laquelle la couche adhésive (2) et le conduit (4) sont couverts par une ou plusieurs feuilles de couverture revêtues d'un antiadhésif.

10. Feuille adhésive selon l'une quelconque des revendications précédentes, dans laquelle ledit conduit (4) comprend un tube imperméable au liquide et au gaz s'étendant transversalement à travers ladite première face de ladite couche support (1).

11. Feuille adhésive selon l'une quelconque des revendications précédentes, dans laquelle ledit conduit (4) comprend un tube perméable au liquide s'étendant transversalement à travers ladite première face de ladite couche support (1).

12. Feuille adhésive selon la revendication 11, dans laquelle ledit tube perméable au liquide comprend un tube perforé.

13. Feuille adhésive selon l'une quelconque des revendications 10 à 12, dans laquelle ledit tube s'étend transversalement à travers ladite première face de ladite couche support (1) d'une manière arquée, sinueuse ou ramifiée.

14. Feuille adhésive selon l'une quelconque des revendications 10 à 13, comprenant un réseau de tubes interconnectés s'étendant transversalement à travers ladite première face de ladite couche support (1).

15. Feuille adhésive comprenant : une couche support (20) ; une couche d'adhésif (21) appliquée à une première face de la couche support (20) pour faire adhérer la feuille adhésive à un substrat ; et un réservoir allongé (22) contenant un agent de libération de liquide placé dans ou sur ladite première face de la couche support (20), dans laquelle le réservoir (22) peut être rompu pour libérer le liquide de celui-ci dans la couche adhésive pendant que la couche adhésive est collée à la surface pour aider à l'enlèvement de la feuille adhésive de la surface.

16. Feuille adhésive selon la revendication 15, dans laquelle le réservoir (22) est muni d'une bande de décollement (23) s'étendant à l'extérieur de la couche support pour libérer le liquide du réservoir lorsque l'on tire la bande de décollement.

17. Ruban adhésif médical destiné à la fixation sur la peau humaine, comprenant une feuille adhésive selon l'une quelconque des revendications 1 à 16.

18. Pansement comprenant une feuille adhésive selon l'une quelconque des revendications 1 à 16.

19. Pansement selon la revendication 18, comprenant en outre une couche de matériau absorbant (3) déposée sur une partie de ladite première face de ladite couche support (1).

20. Pansement selon la revendication 18, dans lequel la couche support (1) comporte une marge revêtue d'adhésif (5) autour de tous les côtés de la couche de matériau absorbant (3).

21. Timbre pour l'administration transdermique d'un médicament comprenant un réservoir du médicament et une feuille adhésive selon l'une quelconque des revendications 1 à 16.
